(19) **Europäisches Patentamt / European Patent Office / Office européen des brevets**

(11) **EP 2 146 680 B1**

(12) **EUROPEAN PATENT SPECIFICATION**

(45) Date of publication and mention
of the grant of the patent:
**19.10.2011 Bulletin 2011/42**

(21) Application number: **08750080.7**

(22) Date of filing: **06.05.2008**

(51) Int Cl.:
*A61K 8/06* (2006.01)     *A61K 8/26* (2006.01)
*A61K 8/73* (2006.01)     *A61Q 19/00* (2006.01)

(86) International application number:
**PCT/EP2008/055528**

(87) International publication number:
**WO 2008/138805 (20.11.2008 Gazette 2008/47)**

(54) **COSMETIC COMPOSITION COMPRISING AN INULIN DERIVATIVE IN THE FORM OF AN EMULSION EXPANDED IN VOLUME**

KOSMETISCHE ZUSAMMENSETZUNG MIT EINEM INULINDERIVAT IN FORM EINER VOLUMSEXPANDIERTEN EMULSION

COMPOSITION COSMÉTIQUE COMPRENANT UN DÉRIVÉ DE L'INULINE SOUS LA FORME D'UNE ÉMULSION EXPANSÉE EN VOLUME

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **10.05.2007   FR 0754958
10.05.2007   FR 0754959
10.05.2007   FR 0754960
16.05.2007   US 938194 P
16.05.2007   US 938196 P
16.05.2007   US 938195 P**

(43) Date of publication of application:
**27.01.2010   Bulletin 2010/04**

(73) Proprietor: **L'Oréal
92665 Asnieres-sur-Seine (FR)**

(72) Inventors:
• **FELTIN, Charlotte
F-75017 Paris (FR)**
• **DOP, Florence
F-91190 Villiers le Bâcle (FR)**

(74) Representative: **Leonard, Armelle et al
L'Oréal
D.I.P.I.
25-29, Quai Aulagnier
92665 Asnières-sur-Seine Cedex (FR)**

(56) References cited:
**EP-A- 1 174 118     WO-A-2007/013484**

• **DATABASE WPI Week 200756 Thomson Scientific, London, GB; AN 2007-575598 XP002491384 & JP 2007 106714 A (SHISEIDO CO LTD) 26 April 2007 (2007-04-26)**
• **TADROS T F ET AL: "Stabilisation of emulsions using hydrophobically modified inulin (polyfructose)" COLLOIDS AND SURFACES. A, PHYSICACHEMICAL AND ENGINEERING ASPECTS, ELSEVIER, AMSTERDAM, NL, vol. 250, no. 1-3, 10 December 2004 (2004-12-10), pages 133-140, XP004675405 ISSN: 0927-7757**

**Description**

A subject-matter of the present invention is a composition expanded in volume, intended for caring for and/or making up keratinous substances and its use, especially as a foundation.

Cosmetic compositions are generally provided in the form of solutions, gels, more or less fluid creams or loose or compact powders. In point of fact, users of products for caring for or making up the skin are increasingly looking for products which are pleasant to use and which have a novel texture.

Thus, the proposal has already been made to introduce a gas, generally air, into cosmetic compositions in order to confer thereon a light texture and to give them the appearance of a foam. This is known as expanding in volume. The foams are assessed for their light textures and the comfort which they provide on application and during the day.

Cosmetic compositions of this type are generally provided in the form of temporary foams produced immediately before use.

They involve either aerosol products dispensed from a pressurized container using a propellant gas, and thus forming a foam, or compositions dispensed from a container by means of a mechanical pump connected to a dispensing head. These packagings exhibit the disadvantage of being fairly expensive.

In addition, compositions expanded in volume immediately before use are not always completely satisfactory, on the one hand in terms of quality of expansion in volume and, on the other hand, of durability over time of the expansion in volume. In the majority of cases, the foam appearance generally generated at the time of the dispensing is very rapidly altered in a detrimental fashion: the foam has a very rapid tendency to subside.

This instability of compositions expanded in volume does not make them very suitable for packaging in jars.

There thus exists a need for cosmetic compositions expanded in volume which can be packaged both in an aerosol and in a jar and which exhibit a degree of expansion in volume which is stable over time.

There thus exists a need for cosmetic compositions exhibiting a sufficiently high viscosity to make possible satisfactory handling of the product, in particular when it is applied, but having a viscosity which can be lowered during the initial manufacture thereof in order to facilitate the production thereof.

The Inventors have shown that it is possible to obtain such a composition by introducing at least one specific hydrophobically modified polysaccharide into an oil-in-water emulsion.

In particular, the compositions according to the invention are provided in the form of a composition expanded in volume or intended to be expanded in volume. The term "composition expanded in volume" is understood to mean, within the meaning of the present patent application, a composition into which a gas has been introduced in order to confer thereon the appearance of a foam, in particular in order to form, within the composition, gas cells separated by thin strips of base composition and thus to confer thereon a foam texture.

Specifically, in order to be expanded in volume, the emulsions have to exhibit a sufficiently low viscosity during the stage of incorporation of the gas in the composition. They must also comprise gelling agents in order for, once expanded in volume, the foam to exhibit good stability.

A subject-matter of the invention, according to a first aspect, is a cosmetic composition expanded in volume, in the form of an oil-in-water emulsion, comprising an internal fatty phase and an external aqueous phase comprising at least one inulin modified by hydrophobic chains and at least two gelling agents for the aqueous phase, one of the two gelling agents being chosen from unmodified clays, in particular montmorillonites, hectorites, smectites, bentones or laponites, or cellulose polymers, such as hydroxyethylcellulose, hydroxypropylcellulose, methylcellulose, ethylhydroxyethylcellulose, carboxymethylcellulose and quaternized cellulose derivatives, and the other gelling agent being chosen from gums arabic, guar gum, xanthan derivatives, karaya gum or locust bean gum, the said internal fatty phase being present in a content ranging from 5 to 50% by weight.

An additional subject-matter of the invention is a method for making up and/or caring for keratinous substances, comprising the application, to the said keratinous substances, of at least one composition as described above.

Hydrophobically modified polysaccharide

The composition according to the invention comprises at least one hydrophobically modified polysaccharide.

In particular, the polysaccharide used in the present invention is chosen from fructans. Fructans or fructosans are oligosaccharides or polysaccharides comprising a sequence of anhydrofructose units optionally in combination with one or more saccharide residues other than fructose. Fructans can be linear or branched. Fructans can be products obtained directly from a plant or microbial source or else products having a chain length which has been modified (increased or reduced) by fractionation, synthesis or hydrolysis, in particular under the effect of an enzyme. Fructans generally have a degree of polymerization of from 2 to approximately 1000 and preferably of from 2 to approximately 60.

3 groups of fructans are distinguished. The first group corresponds to products, the fructose units of which are for the most part bonded via β-2-1 bonds. These are essentially linear fructans, such as inulins.

The second group also corresponds to linear fructoses but the fructose units are essentially bonded via β-2-6 bonds. These products are levans.

The third group corresponds to mixed fructans, that is to say having β-2-6 and β-2-1 sequences. These are essentially branched fructans, such as graminans.

The fructans used in the compositions according to the invention are inulins. The inulin can be obtained, for example, from endive, dahlia or Jerusalem artichoke. Preferably, the inulin used in the composition according to the invention is obtained, for example, from endive.

The polysaccharides, in particular inulins, used in the compositions according to the invention are hydrophobically modified. In particular, they are obtained by grafting hydrophobic chains to the hydrophilic backbone of the fructan.

The hydrophobic chains capable of being grafted to the main chain of the fructan can in particular be saturated or unsaturated and linear or branched hydrocarbon chains having from 1 to 50 carbon atoms, such as alkyl, arylalkyl, alkylaryl or alkylene groups; divalent cycloaliphatic groups or organopolysiloxane chains. These hydrocarbon or organopolysiloxane chains can in particular comprise one or more ester, amide, urethane, carbamate, thiocarbamate, urea, thiourea and/or sulphonamide functional groups, such as, in particular, methylenedicyclohexyl and isophorone; or dilvant aromatic groups, such as phenylene.

In particular, the polysaccharide, in particular inulin, exhibits a degree of polymerization of from 2 to approximately 1000 and preferably of from 2 top approximately 60, and a degree of substitution of less than 2 on the basis of one fructose unit.

According to a preferred embodiment, the hydrophobic chains exhibit at least one alkylcarbamate group of formula R-NH-CO- in which R is an alkyl group having from 1 to 22 carbon atoms.

According to a more preferred embodiment, the hydrophobic chains are laurylcarbamate groups.

Mention may in particular be made, by way of illustration and without limitation of the hydrophobically modified inulins which can be used in the compositions according to the invention, of stearoyl inulin, such as those sold under the names Lifidrem INST by Engelhard and Rheopearl INS by Ciba; palmitoyl inulin; undecylenoyl inulin, such as those sold under the names Lifidrem INUK and Lifidrem INUM by Engelhard; and inulin laurylcarbamate, such as that sold under the name Inutec SP1 by Orafti.

In particular, the hydrophobically modified polysaccharide is an inulin grafted with laurylcarbamate, resulting in particular from the reaction of lauryl isocyanate with an inulin, in particular resulting from endive. Mention may in particular be made, as example of these compounds, of the product sold under the name Inutec SP1 by Orafti.

It is considered that the viscosity is significantly lowered within the meaning of the present patent application when the variation in viscosity between the composition without polysaccharide and the composition with polysaccharide is at least 5%.

The hydrophobically treated polysaccharide, in particular inulin, is present in the composition according to the invention in a content ranging from 0.01 to 20% by weight, with respect to the total weight of the composition, preferably from 0.05 to 15% by weight, more preferably from 0.1 to 5% by weight and more preferably still from 0.1 to 1% by weight.

**Fatty phase**

The composition according to the invention comprises at least one internal fatty phase comprising an oily phase.

According to a specific embodiment, the internal fatty phase is present in the composition according to the invention in a content ranging from 5 to 50% by weight, with respect to the total weight of the composition, preferably from 10 to 40% by weight and more preferably from 15 to 30% by weight.

The term "oily phase" is understood to mean the combined oils present in the composition.

The oily phase can comprise at least one oil chosen from volatile oils, nonvolatile oils and their mixture.

According to a preferred embodiment, the composition according to the invention can comprise at least one nonvolatile oil.

The term "nonvolatile oil" is understood to mean an oil which remains on the skin at ambient temperature and atmospheric pressure for at least several hours and which has in particular a vapour pressure of less than 0.13 Pa (0.01 mmHg) at ambient temperature (25°C).

These nonvolatile oils can be hydrocarbon oils, in particular of animal or vegetable origin, silicone oils or their mixtures. The term "hydrocarbon oil" is understood to mean an oil comprising mainly hydrogen and carbon atoms and optionally oxygen, nitrogen, sulphur and/or phosphorus atoms.

The nonvolatile oils can in particular be chosen from nonvolatile hydrocarbon oils, if appropriate fluorinated, and/or nonvolatile silicone oils.

Mention may in particular be made, as nonvolatile hydrocarbon oil, of:

- hydrocarbon oils of animal origin,

- hydrocarbon oils of vegetable origin, such as triglycerides composed of esters of fatty acids and of glycerol, the fatty acids of which can have varied chain lengths from $C_4$ to $C_{24}$, it being possible for these chains to be saturated or unsaturated and linear or branched; these oils are in particular triglycerides of heptanoic acid or of octanoic acid or else wheat germ, sunflower, grape seed, sesame, maize, apricot kernel, castor, shea, avocado, olive, soybean, sweet almond, palm, rapeseed, cottonseed, hazelnut, macadamia, jojoba, alfalfa, poppy, pumpkinseed, cucumber, blackcurrant seed, evening primrose, millet, barley, quinoa, rye, safflower, candlenut, passionflower or musk rose oil; shea butter; or also triglycerides of caprylic/capric acids, such as those sold by Stéarineries Dubois or those sold under the names Miglyol 810®, 812® and 818® by Dynamit Nobel,

- synthetic ethers having from 10 to 40 carbon atoms,

- linear or branched hydrocarbons of mineral or synthetic origin, such as liquid petrolatum, polydecenes, hydrogenated polyisobutene, such as Parleam®, squalane, liquid paraffins, and their mixtures,

- synthetic esters, such as the oils of formula $R_1COOR_2$ in which $R_1$ represents the residue of a linear or branched fatty acid comprising from 1 to 40 carbon atoms and $R_2$ represents a hydrocarbon chain, in particular a branched hydrocarbon chain, comprising from 1 to 40 carbon atoms, provided that $R_1 + R_2$ is $\geq 10$, such as, for example Purcellin oil (ketostearyl octanoate), isopropyl myristate, isopropyl palmitate, benzoates of $C_{12}$ to $C_{15}$ alcohols, hexyl laurate, diisopropyl adipate, isononyl isononanoate, isodecyl neopentanoate, 2-ethylhexyl palmitate, isostearyl isostearate, 2-hexyldecyl laurate, 2-octyldecyl palmitate, 2-octyldodecyl myristate, heptanoates, octanoates, decanoates or ricinoleates of alcohols or polyalcohols, such as propylene glycol dioctanoate; hydroxylated esters, such as isostearyl lactate, diisostearyl malate or 2-octyldodecyl lactate; esters of polyols and esters of pentaerythritol,

- fatty alcohols comprising a branched and/or unsaturated carbon chain having from 12 to 26 carbon atoms which are liquid at ambient temperature, such as octyldodecanol, isostearyl alcohol, oleyl alcohol, 2-hexyldecanol, 2-butyloctanol and 2-undecylpentadecanol,

- higher fatty acids, such as oleic acid, linoleic acid, linolenic acid and their mixtures.

The nonvolatile silicone oils which can be used in the composition according to the invention can be polydimethylsiloxanes (PDMSs) which are nonvolatile, polydimethylsiloxanes comprising pendent alkyl or alkoxy groups and/or alkyl or alkoxy groups at the ends of the silicone chain, groups each having from 2 to 24 carbon atoms, phenylated silicones, such as phenyl trimethicones, phenyl dimethicones, phenyl(trimethylsiloxy)diphenylsiloxanes, diphenyl dimethicones or diphenyl(methyldiphenyl)trisiloxanes, and their mixtures.

According to a preferred embodiment, the composition according to the invention can comprise at least two different nonvolatile oils.

More preferably, the composition according to the invention can comprise at least two different nonvolatile oils chosen from hydrocarbon oils of vegetable origin, such as triglycerides composed of esters of fatty acids and of glycerol, the fatty acids of which can have varied chain lengths from $C_4$ to $C_{24}$, it being possible for these chains to be saturated or unsaturated and linear or branched; these oils are in particular triglycerides of heptanoic acid or of octanoic acid or else wheat germ, sunflower, grape seed, sesame, maize, apricot kernel, castor, shea, avocado, olive, soybean, sweet almond, palm, rapeseed, cottonseed, hazelnut, macadamia, jojoba, alfalfa, poppy, pumpkinseed, cucumber, blackcurrant seed, evening primrose, millet, barley, quinoa, rye, safflower, candlenut, passionflower or musk rose oil; shea butter; or also triglycerides of caprylic/capric acids, such as those sold by Stéarineries Dubois or those sold under the names Miglyol 810®, 812® and 818® by Dynamit Nobel, and their mixture.

In particular, the nonvolatile oil(s) can be chosen from triglycerides of caprylic/capric acids, jojoba oil and their mixture, such as are described above.

The nonvolatile oils can be present in the composition according to the invention in a content ranging from 1% to 60% by weight, with respect to the total weight of the composition, preferably ranging from 3% to 50% by weight, preferentially ranging from 5% to 35% by weight and more preferably ranging from 10% to 30% by weight.

The composition according to the invention can additionally comprise at least one volatile oil.

The term "volatile oil" is understood to mean, within the meaning of the invention, any oil capable of evaporating on contact with the skin, at ambient temperature and atmospheric pressure. The volatile oils of the invention are volatile cosmetic oils which are liquid at ambient temperature and which have a nonzero vapour pressure, at ambient temperature and atmospheric pressure, ranging in particular from 0.13 Pa to 40 000 Pa (0.001 to 300 mmHg) and preferably ranging from 1.3 to 1300 Pa (0.01 to 10 mmHg).

The volatile oil can be chosen from volatile hydrocarbon oils, volatile silicone oils, volatile fluorinated oils and their mixtures.

According to a preferred embodiment, the volatile oil is chosen from hydrocarbon oils.

The term "hydrocarbon oil" is understood to mean an oil comprising mainly hydrogen and carbon atoms and optionally oxygen, nitrogen, sulphur and/or phosphorus atoms.

Volatile hydrocarbon oils can be chosen from hydrocarbon oils having from 8 to 16 carbon atoms, in particular branched $C_8$-$C_{16}$ alkanes, such as $C_8$-$C_{16}$ isoalkanes of petroleum origin (also known as isoparaffins), such as isodecane (also known as 2,2,4,4,6-pentamethylheptane) or isohexadecane, for example the oils sold under the Isopar® or Permethyl® trade names.

Use may also be made, as volatile oils, of volatile silicones, such as, for example, volatile linear or cyclic silicone oils, in particular those having a viscosity $\leq$ 5 centistokes ($5 \times 10^{-6}$ m$^2$/s) and having in particular from 2 to 10 silicon atoms, preferably from 2 to 7 silicon atoms, these silicones optionally comprising alkyl or alkoxy groups having from 1 to 10 carbon atoms. Mention may in particular be made, as volatile silicone oil which can be used in the invention, of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane and their mixtures.

The volatile fluorinated oil generally does not have a flashpoint.

Mention may be made, as volatile fluorinated oil, of nonafluoroethoxybutane, nonafluoromethoxybutane, decafluoropentane, tetradecafluorohexane, dodecafluoropentane and their mixtures.

The volatile oil can be present in the composition according to the invention at a content ranging from 10% to 70% by weight, with respect to the total weight of the composition, preferably ranging from 10% to 60% by weight, preferentially ranging from 10% to 40% by weight and more preferably ranging from 15% to 30% by weight.

The oily phase of the composition according to the invention is present in a total content ranging from 1% to 60% by weight, with respect to the total weight of the composition, preferably ranging from 3% to 50% by weight, preferentially ranging from 5% to 35% by weight and more preferably ranging from 10% to 30% by weight.

Structuring agent

The composition according to the invention can comprise at least one structuring agent for the liquid fatty phase chosen from waxes, pasty fatty substances, semicrystalline polymers, lipophilic gelling agents and their mixtures.

*Waxes*

The composition according to the invention can comprise at least one wax in a content which can range from 1 to 15% by weight, with respect to the total weight of the composition, preferably from 2 to 12% by weight and better still from 4 to 9% by weight.

The term "wax" is understood to mean, within the meaning of the present invention, a lipophilic fatty compound which is solid at ambient temperature (25°C) and atmospheric pressure (760 mmHg, i.e. 10$^5$ Pa), which exhibits a reversible solid/liquid change in state and which has in particular a melting point of greater than or equal to 30°C, in particular of greater than or equal to 45°C, and which can range up to 250°C, in particular up to 230°C and especially up to 120°C.

On bringing the wax to its melting point, it is possible to render it miscible with oils and to form a microscopically homogeneous mixture but, on reestablishing the temperature of the mixture at ambient temperature, recrystallization of the wax in the oils of the mixture is obtained.

The melting point values correspond, according to the invention, to the melting peak measured using a differential scanning calorimeter (DSC), for example the calorimeter sold under the name DSC 30 by Metler, with a rise in temperature of 5 or 10°C per minute.

The waxes, within the meaning of the invention, can be those generally used in cosmetics or dermatological fields. They can in particular be hydrocarbon, silicone and/or fluorinated waxes, optionally comprising ester or hydroxyl functional groups. They can also be of natural or synthetic origin.

Mention may in particular be made, by way of illustration and without limitation of these waxes, of:

- beeswax, lanolin wax, and Chinese insect waxes; rice wax, carnauba wax, candelilla wax, ouricury wax, cork fibre wax, sugarcane wax, Japan wax and sumac wax; montan wax, microcrystalline waxes, paraffin waxes, ozokerites, ceresin wax, lignite wax, polyethylene waxes, waxes obtained by the Fischer-Tropsch synthesis, and fatty acid esters and glycerides which are solid at 40°C and in particular at more than 45°C,
- waxes obtained by catalytic hydrogenation of animal or vegetable oils having linear or branched $C_8$-$C_{32}$ fatty chains, in particular hydrogenated jojoba oil, hydrogenated sunflower oil, hydrogenated castor oil, hydrogenated coconut oil and hydrogenated lanolin oil,
- silicone waxes or fluorinated waxes, and
- their mixtures.

According to a specific alternative form of the invention, the wax can be chosen from carnauba waxes, microcrystalline waxes and their mixtures.

*Pasty fatty substances*

The term "pasty fatty substances" is understood to mean a lipophilic fatty compound comprising, at a temperature of 23°C, a liquid fraction and a solid fraction.

The said pasty compound preferably has a hardness at 20°C ranging from 0.001 to 0.5 MPa, preferably from 0.002 to 0.4 MPa.

The hardness is measured according to a method of penetration of a probe into a sample of compound and in particular using a texture analyser (for example, the TA-XT2i from Rheo) equipped with a stainless steel cylinder with a diameter of 2 mm. The hardness measurement is carried out at 20°C at the centre of 5 samples. The cylinder is introduced into each sample at a pre-rate of 1 mm/s and then at a measuring rate of 0.1 mm/s, the depth of penetration being 0.3 mm. The value recorded for the hardness is that of the maximum peak.

The liquid fraction of the pasty compound measured at 23°C preferably represents 9 to 97% by weight of the compound. This liquid fraction at 23°C preferably represents between 15 and 85%, more preferably between 40 and 85%, by weight. The liquid fraction by weight of the pasty compound at 23°C is equal to the ratio of the enthalpy of fusion consumed at 23°C to the enthalpy of fusion of the pasty compound.

The enthalpy of fusion of the pasty compound is the enthalpy consumed by the compound to change from the solid state to the liquid state. The pasty compound is "in the solid state" when the whole of its mass is in the crystalline solid form. The pasty compound is "in the liquid state" when the whole of its mass is in the liquid form.

The enthalpy of fusion of the pasty compound is equal to the area under the curve of the thermogram obtained using a differential scanning calorimeter (DSC), such as the calorimeter sold under the name MDSC 2920 by TA Instrument, with a rise in temperature of 5 or 10°C per minute, according to the standard ISO 11357-3:1999. The enthalpy of fusion of the pasty compound is the amount of energy necessary to change the compound from the solid state to the liquid state. It is expressed in J/g.

The enthalpy of fusion consumed at 23°C is the amount of energy absorbed by the sample to change from the solid state to the state which it exhibits at 23°C, composed of a liquid fraction and of a solid fraction. The liquid fraction of the pasty compound measured at 32°C preferably represents from 30 to 100% by weight of the compound, preferably from 80 to 100%, more preferably from 90 to 100% by weight of the compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the enthalpy of fusion consumed at 32°C to the enthalpy of fusion of the pasty compound. The enthalpy of fusion consumed at 32°C is calculated in the same way as the enthalpy of fusion consumed at 23°C.

The pasty substances are generally hydrocarbon compounds, such as lanolins and their derivatives, or also PDMSs.

*Semicrystalline polymers*

The term "polymer" is understood to mean compounds comprising at least two repeat units, preferably at least 3 repeat units and more especially at least 10 repeat units. The term "semicrystalline polymer" is understood to mean polymers comprising a crystallizable part, a crystallizable pendent chain or a crystallizable block in the backbone, and an amorphous part in the backbone and exhibiting a first-order reversible phase change temperature, in particular a melting point (solid-liquid transition). When the crystallizable part is in the form of a crystallizable block of the polymer backbone, the amorphous part of the polymer is in the form of an amorphous block; the semicrystalline polymer is in this case a block copolymer, for example of the diblock, triblock or multiblock type, comprising at least one crystallizable block and at least one amorphous block. The term "block" is understood to mean generally at least 5 identical repeat units. The crystallizable block or blocks are then different in chemical nature from the amorphous block or blocks.

The semicrystalline polymer has a melting point of greater than or equal to 30°C (in particular ranging from 30°C to 80°C), preferably ranging from 30°C to 60°C. This melting point is a first-order change in state temperature.

This melting point can be measured by any known method, in particular using a differential scanning calorimeter (DSC).

Advantageously, the semicrystalline polymer or polymers to which the invention applies exhibit a number-average molecular weight of greater than or equal to 1000. Advantageously, the semicrystalline polymer or polymers of the composition of the invention have a number-average molecular weight Mn ranging from 2000 to 800 000, preferably from 3000 to 500 000, better still from 4000 to 150 000, in particular of less than 100 000, and better still from 4000 to 99 000. Preferably, they exhibit a number-average molecular weight of greater than 5600, for example ranging from 5700 to 99 000. The term "crystallizable chain or block" is understood to mean, within the meaning of the invention, a chain or block which, if it were alone, would change reversibly from the amorphous state to the crystalline state according to whether the temperature is above or below the melting point. A chain within the meaning of the invention is a group of atoms which is pendent or lateral with respect to the backbone of the polymer. A block is a group of atoms belonging to the backbone, a group constituting one of the repeat units of the polymer. Advantageously, the "crystallizable pendent chain" can be a chain comprising at least 6 carbon atoms.

The semicrystalline polymer can be chosen from block copolymers comprising at least one crystallizable block and at least one amorphous block, homopolymers and copolymers carrying at least one crystallizable side chain per repeat unit, or their blends.

Such polymers are described, for example, in the document EP 1 396 259.

According to a more specific embodiment of the invention, the polymer results from a monomer comprising a crystallizable chain chosen from saturated $C_{14}$ to $C_{22}$ alkyl (meth)acrylates.

Mention may be made, as specific example of structuring semicrystalline polymer which can be used in the composition according to the invention, of the Intelimer® products from Landec described in the brochure "Intelimer® polymers", Landec IP22. These polymers are in the solid form at ambient temperature (25°C) .

*Lipophilic gelling agents*

The gelling agents which can be used in the compositions according to the invention can be polymeric or molecular and organic or inorganic lipophilic gelling agents.

Mention may be made, as inorganic lipophilic gelling agent, of optionally modified clays, such as hectorites modified by a $C_{10}$ to $C_{22}$ fatty acid ammonium chloride, such as hectorite modified by distearyldimethylammonium chloride, such as, for example, that sold under the name of Bentone 38V® by Elementis.

Mention may also be made of pyrogenic silica optionally treated hydrophobically at the surface, the size of the particles of which is less than 1 μm. This is because it is possible to chemically modify the surface of the silica by chemical reaction, resulting in a reduction in the number of silanol groups present at the surface of the silica. It is possible in particular to substitute silanol groups by hydrophobic groups: a hydrophobic silica is then obtained. The hydrophobic groups can be:

- trimethylsiloxyl groups, which are obtained in particular by treatment of pyrogenic silica in the presence of hexamethyldisilazane. Silicas thus treated are named "Silica silylate" according to the CTFA (6th edition, 1995). They are, for example, sold under the references Aerosil R812® by Degussa and Cab-0-Sil TS-530® by Cabot,
- dimethylsilyloxyl or polydimethylsiloxane groups, which are obtained in particular by treatment of pyrogenic silica in the presence of polydimethylsiloxane or dimethyldichlorosilane. Silicas thus treated are named "Silica dimethyl silylate" according to the CTFA (6th edition, 1995). They are, for example, sold under the references Aerosil R972® and Aerosil R974® by Degussa and Cab-0-Sil TS-610® and Cab-O-Sil TS-720® by Cabot.

The hydrophobic pyrogenic silica exhibits in particular a particle size which can be nanometric to micrometric, for example ranging from approximately 5 to 200 nm.

The polymeric organic lipophilic gelling agents are, for example, partially or completely crosslinked organopolysiloxane elastomers of three-dimensional structure, such as those sold under the names of KSG6®, KSG16® and KSG18® by Shin-Etsu, of Trefil E-505C® and Trefil E-506C® by Dow Corning, of Gransil SR-CYC®, SR DMF10®, SR-DC556®, SR 5CYC gel®, SR DMF 10 gel® and SR DC 556 gel® by Grant Industries, of SF 1204® and of JK 113® by General Electric; ethylcellulose, such as that sold under the name Ethocel® by Dow Chemical; polycondensates of polyamide type resulting from the condensation between (α) at least one acid chosen from dicarboxylic acids comprising at least 32 carbon atoms, such as dimeric fatty acids, and (β) an alkylenediamine, in particular ethylenediamine, in which the polyamide polymer comprises at least one end carboxylic acid group esterified or amidated with at least one linear and saturated monoalcohol or one linear and saturated monoamine comprising from 12 to 30 carbon atoms, in particular ethylenediamine/stearyl dilinoleate copolymers, such as that sold under the name Uniclear 100 VG® by Arizona Chemical; silicone polyamides of the polyorganosiloxane type, such as those described in the documents US-A-5 874 069, US-A-5 919 441, US-A-6 051 216 and US-A-5 981 680, such as, for example, those sold under the references Dow Corning 2-8179 and Dow Corning 2-8178 Gellant by Dow Corning; galactomannans comprising from one to six and in particular from two to four hydroxyl groups per monosaccharide which are substituted by a saturated or unsaturated alkyl chain, such as guar gum alkylated by $C_1$ to $C_6$ alkyl chains, in particular $C_1$ to $C_3$ alkyl chains, and their mixtures; block copolymers of "diblock", "triblock" or "radial" type of the polystyrene/polyisoprene or polystyrene/polybutadiene type, such as those sold under the name Luvitol HSB® by BASF, of the polystyrene/copoly(ethylene-propylene) type, such as those sold under the name Kraton® by Shell Chemical Co., or of the polystyrene/copoly-(ethylene-butylene) type, blends of triblock and radial (star) copolymers in isododecane, such as those sold by Penreco under the name Versagel®,

such as, for example, the blend of butylene/ethylene/styrene triblock copolymer and of ethylene/propylene/styrene star copolymer in isododecane (Versagel M 5960).

Mention may also be made, among the lipophilic gelling agents which can be used in the compositions according to the invention, of esters of dextrin and of fatty acid, such as dextrin palmitates, in particular such as those sold under the names Rheopearl TL® or Rheopearl KL® by Chiba Flour.

The structuring agent can be present in the composition according to the invention in a content ranging from 0.1% to 10% by weight, with respect to the total weight of the composition, preferably in a content ranging from 0.5% to 7% by weight.

## Aqueous phase

The composition according to the invention comprises an aqueous phase. The aqueous phase can in particular constitute the external phase of the composition according to the invention.

The composition according to the invention comprises water. The water can be a floral water, such as cornflower water, and/or a mineral water, such as water from Vittel, water from Lucas or water from La Roche-Posay, and/or a thermal water.

The composition according to the invention, in particular the aqueous phase, can also comprise water-miscible organic solvents (miscible at ambient temperature - 25°C), such as, for example, monoalcohols having from 2 to 6 carbon atoms, such as ethanol or isopropanol;

polyols having in particular from 2 to 20 carbon atoms, preferably having from 2 to 10 carbon atoms and preferably having from 2 to 6 carbon atoms, such as glycerol, propylene glycol, butylene glycol, pentylene glycol, hexylene glycol, dipropylene glycol or diethylene glycol;

glycol ethers (having in particular from 3 to 16 carbon atoms), such as mono-, di- or tripropylene glycol $(C_1-C_4)$alkyl ethers or mono-, di- or triethylene glycol $(C_1-C_4)$alkyl ethers, and their mixtures.

The liquid fraction of the aqueous phase comprises in particular water and water-miscible organic solvents.

The aqueous phase can additionally comprise stabilizing agents, for example sodium chloride, magnesium dichloride and magnesium sulphate.

Preferably, the aqueous phase can be present in the composition according to the invention in a content ranging from 1% to 65% by weight, with respect to the total weight of the composition, preferably ranging from 10% to 60% by weight and preferentially ranging from 20% to 55% by weight.

### Hydrophilic gelling agent

The composition according to the invention comprises at least two hydrophilic gelling agents in a content, on a dry basis, which can range from 0.01% to 30% by weight, with respect to the total weight of the composition, preferably from 0.05% to 20% by weight and more preferably from 0.1% to 10% by weight.

In particular, the composition according to the invention comprises at least two hydrophilic gelling agents, one of the two gelling agents being chosen from unmodified clays, in particular montmorillonites, hectorites, smectites, bentones or laponites, or cellulose polymers, such as hydroxyethylcellulose, hydroxypropylcellulose, methylcellulose, ethylhydroxyethylcellulose, carboxymethylcellulose and quaternized cellulose derivatives, and the other gelling agent being chosen from gums arabic, guar gum, xanthan derivatives, karaya gum or locust bean gum.

According to a particularly preferred form, the composition according to the invention can comprise a mixture of xanthan gum and of smectite.

In addition to the two gelling agents described above, the composition according to the invention can comprise another hydrophilic gelling agent. The additional hydrophilic gelling agent which can be used in the compositions according to the invention can be chosen from:

homo- or copolymers of acrylic acid or methacrylic acid or their salts and their esters and in particular the products sold under the names Versicol F® or Versicol K® by Allied Colloid, Ultrahold 8® by Ciba-Geigy, poly(acrylic acid)s of Synthalen K type,

copolymers of acrylic acid and of acrylamide, sold in the form of their sodium salts under the Reten® names by Hercules, poly(sodium methacrylate), sold under the name Darvan No. 7® by Vanderbilt, sodium salts of poly(hydroxycarboxylic acid)s, sold under the name Hydagen F® by Henkel,

copolymers of poly(acrylic acid)s and of alkyl acrylates of Pemulen type,

AMPS (poly(acrylamidomethylpropanesulphonic acid) partially neutralized with aqueous ammonia and highly crosslinked), sold by Clariant,

AMPS/acrylamide copolymers of Sepigel® or Simugel® type, sold by Seppic, and

copolymers of AMPS and of alkyl methacrylates which are polyoxyethylenated (crosslinked or noncrosslinked),

proteins, such as proteins of plant origin, such as wheat or soya proteins; proteins of animal origin, such as keratins, for example keratin hydrolysates and sulphonic keratins;

acrylic polymers or copolymers, such as polyacrylates or polymethacrylates;

vinyl polymers, such as polyvinylpyrrolidones, copolymers of methyl vinyl ether and of malic anhydride, the copolymer of vinyl acetate and of crotonic acid, copolymers of vinylpyrrolidone and of vinyl acetate, copolymers of vinylpyrrolidone and of caprolactam, or poly(vinyl alcohol);

optionally modified polymers of natural origin, such as:

alginates and carrageenans (the alginates being used preferably in the presence of salts, such as, for example, calcium chloride);

glycoaminoglycans, hyaluronic acid and its derivatives;

shellac resin, gum sandarac, dammars, elemis or copals;

deoxyribonucleic acid;

mucopolysaccharides, such as chondroitin sulphates,

and their mixtures.

Some of these water-soluble gelling agents can also act as film-forming polymers.

*Emulsifying system*

The compositions according to the invention can comprise emulsifying surface-active agents.

According to the invention, use is generally made of an emulsifier appropriately chosen in order to obtain an oil-in-water emulsion. Use may in particular be made of an emulsifier having, at 25°C, an HLB (hydrophiliclipophilic balance) within the meaning of Griffin of greater than or equal to 8.

The HLB value according to Griffin is defined in J. Soc. Cosm. Chem., 1954 (volume 5), pages 249-256.

These surface-active agents can be chosen from nonionic, anionic, cationic or amphoteric surface-active agents or also surface-active emulsifiers. Reference may be made to the document "Encyclopedia of Chemical Technology", Kirk-Othmer, volume 22, pp. 333-432, 3rd edition, 1979, Wiley, for the definition of the properties and functions (emulsifying) of surfactants, in particular pp. 347-377 of this reference for the anionic, amphoteric and nonionic surfactants.

The surfactants preferably used in the composition according to the invention are chosen from:

a) nonionic surface-active agents with an HLB of greater than or equal to 8 to 25°C, used alone or as a mixture; mention may in particular be made of:

fatty alcohols, in particular $C_8$-$C_{24}$ alcohols and preferably $C_{12}$-$C_{18}$ alcohols, such as cetylstearyl alcohol;

oxyethylenated and/or oxypropylenated ethers (which can comprise from 1 to 150 oxyethylene and/or oxypropylene groups) of glycerol;

oxyethylenated and/or oxypropylenated ethers (which can comprise from 1 to 150 oxyethylene and/or oxypropylene groups) of fatty alcohols (in particular of $C_8$-$C_{24}$ and preferably $C_{12}$-$C_{18}$ alcohols), such as the oxyethylenated ether of stearyl alcohol comprising 20 oxyethylene groups (CTFA name "Steareth-20"), such as Brij 78, sold by Uniquema, the oxyethylenated ether of cetearyl alcohol comprising 30 oxyethylene groups (CTFA name "Ceteareth-30") and the oxyethylenated ether of the mixture of $C_{12}$-$C_{15}$ fatty alcohols comprising 7 oxyethylene groups (CTFA name "C12-15 Pareth-7") sold under the name Neodol 25-7® by Shell Chemicals,

esters of fatty acid (in particular of $C_8$-$C_{24}$ and preferably $C_{16}$-$C_{22}$ acid) and of polyethylene glycol (which can comprise from 1 to 150 ethylene glycol units), such as PEG-50 stearate and PEG-40 monostearate, sold under the name Myrj 52P® by ICI Uniquema,

esters of fatty acid (in particular of $C_8$-$C_{24}$ and preferably $C_{16}$-$C_{22}$ acid) and of the oxyethylenated and/or oxypropylenated glycerol ethers (which can comprise from 1 to 150 oxyethylene and/or oxypropylene groups), such as PEG-200 glyceryl monostearate, sold under the name Simulsol 220 TM® by Seppic; polyethoxylated glyceryl stearate comprising 30 ethylene oxide groups, such as the product Tagat S® sold by Goldschmidt, polyethoxylated glyceryl oleate comprising 30 ethylene oxide groups, such as the product Tagat O® sold by Goldschmidt, polyethoxylated glyceryl cocoate comprising 30 ethylene oxide groups, such as the product Varionic LI 13® sold by Sherex, polyethoxylated glyceryl isostearate comprising 30 ethylene oxide groups, such as the product Tagat L® sold by Goldschmidt, and polyethoxylated glyceryl laurate comprising 30 ethylene oxide groups, such as the product Tagat I® from Goldschmidt,

esters of fatty acid (in particular of $C_8$-$C_{24}$ and preferably $C_{16}$-$C_{22}$ acid) and of the oxyethylenated and/or oxypropylenated sorbitol ethers (which can comprise from 1 to 150 oxyethylene and/or oxypropylene groups), such as polysorbate 60, sold under the name Tween 60® by Uniquema,

dimethicone copolyol, such as that sold under the name Q2-5220® by Dow Corning,
dimethicone copolyol benzoate (Finsolv SLB 101® and 201® from Fintex),
copolymers of propylene oxide and of ethylene oxide, also known as EO/PO polycondensates,
and their mixtures.

The EO/PO polycondensates are more particularly copolymers consisting of polyethylene glycol and polypropylene glycol blocks, such as, for example, polyethylene glycol/polypropylene glycol/polyethylene glycol triblock polycondensates. These triblock polycondensates have, for example, the following chemical structure:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

in which formula a ranges from 2 to 120 and b ranges from 1 to 100.

The EO/PO polycondensate preferably has a weight-average molecular weight ranging from 1000 to 15 000 and better still ranging from 2000 to 13 000. Advantageously, the said EO/PO polycondensate has a cloud point, at 10 g/l in distilled water, of greater than or equal to 20°C, preferably of greater than or equal to 60°C. The cloud temperature is measured according to Standard ISO 1065. Mention may be made, as EO/PO polycondensate which can be used according to the invention, of the polyethylene glycol/polypropylene glycol/polyethylene glycol triblock polycondensates sold under the Synperonic® names, such as Synperonic PE/L44® and Synperonic PE/F127®, by ICI.

b) nonionic surface-active agents with an HLB of less than 8 at 25°C, optionally in combination with one or more nonionic surface-active agents with an HLB of greater than 8 at 25°C, such as mentioned above, such as:

esters and ethers of monosaccharides, such as sucrose stearates, sucrose cocoates, sorbitan stearates and their mixtures, in particular those sold under the name Arlatone 2121® by ICI or Span 65V by Uniquema;
esters of fatty acids (in particular of $C_8$-$C_{24}$ and preferably $C_{16}$-$C_{22}$ acid) and of polyol, in particular of glycerol or of sorbitol, such as glyceryl stearate, such as, for example, those sold under the name Tegin M® by Goldschmidt, the mixture of glycerol mono- and distearate sold under the name of "Geleol" by Gattefossé, glyceryl laurate, such as the product sold under the name Imwitor 312® by Hüls, polyglyceryl-2 stearate, sorbitan tristearate or glyceryl ricinoleate;
oxyethylenated and/or oxypropylenated ethers, such as the oxyethylenated ether of stearyl alcohol comprising 2 oxyethylene groups (CTFA name "Steareth-2"), such as Brij 72, sold by Uniquema;
the cyclomethicone/dimethicone copolyol mixture sold under the name Q2-3225C® by Dow Corning,

c) anionic surfactants, such as:

salts of $C_{16}$-$C_{30}$ fatty acids, in particular those deriving from amines, such as triethanolamine stearate;
salts of polyoxyethylenated fatty acids, in particular those deriving from amines or the alkali metal salts, and their mixtures;
phosphoric esters and their salts, such as "DEA oleth-10 phosphate" (Crodafos N 10N from Croda) or monopotassium monocetyl phosphate (Amphisol K from Givaudan or Arlatone MAP 160K from Uniquema);
sulphosuccinates, such as "Disodium PEG-5 citrate lauryl sulphosuccinate" and "Disodium ricinoleamido MEA sulphosuccinate";
alkyl ether sulphates, such as sodium lauryl ether sulphate;
isethionates;
acylglutamates, such as "Disodium hydrogenated tallow glutamate" (Amisoft HS-21 R®, sold by Ajinomoto), and their mixtures.

Mention may in particular be made, by way of representation of cationic surfactants, of:

- alkyl imidazolidiniums, such as isostearyl ethylimidonium ethosulphate,
- ammonium salts, such as N,N,N-trimethyl-1-docosanaminium chloride (behentrimonium chloride).

The compositions according to the invention can also comprise one or more amphoteric surfactants, such as N-acylamino acids, for example N-acylaminoacetates and disodium cocoamphodiacetate, and amine oxides, such as stearamine oxide, or also silicone surfactants, such as dimethicone copolyol phosphates, such as that sold under the name Pecosil PS 100® by Phoenix Chemical.
According to a preferred embodiment, the composition according to the invention can comprise at least two different surfactants.

In particular, the preferred surfactants can be chosen from nonionic surface-active agents with an HLB of less than 8 at 25°C.

According to a more preferred embodiment, the surfactants can be chosen from esters of fatty acids (in particular of $C_8$-$C_{24}$ and preferably $C_{16}$-$C_{22}$ acid) and of polyol, in particular of glycerol or of sorbitol, such as glyceryl stearate, such as, for example, those sold under the name Tegin M® by Goldschmidt, the mixture of glycerol mono- and distearate and of potassium stearate, such as, for example, those sold under the name Tegin Pellets® by Goldschmidt, the mixture of glycerol mono- and distearate sold under the name "Geleol" by Gattefossé, glyceryl laurate, such as the product sold under the name Imwitor 312® by Hüls, polyglyceryl-2 stearate, sorbitan tristearate or glyceryl ricinoleate.

According to an even more preferred embodiment, the composition can comprise a mixture of glycerol mono- and distearate and of potassium stearate.

The emulsifying surface-active agents are present in particular in a proportion ranging from 0 .1 to 20% by weight, with respect to the total weight of the composition, preferably from 0.5 to 15% by weight and more preferably from 1 to 10% by weight.

## Pulverulent phase

The composition according to the invention can comprise a pulverulent phase in a content which can range from 1 to 30% by weight, with respect to the total weight of the composition, preferably from 5 to 25% by weight.

The pulverulent phase can comprise pigments, fillers and/or pearlescent agents.

The term "pigments" should be understood as meaning inorganic or organic particles which are insoluble in the liquid organic phase and which are intended to colour and/or opacify the composition.

The pigments can be inorganic or organic pigments. Use may be made, as pigments, of metal oxides, such as iron oxides (in particular those yellow, red, brown or black in colour), titanium dioxides, cerium oxide, zirconium oxide, chromium oxide, manganese violet, ultramarine blue, Prussian blue, ferric blue, and their mixtures. Use is preferably made of iron oxide or titanium dioxide pigments.

The pigments can be treated with a hydrophobic agent in order to render them compatible with the organic phase of the composition. The hydrophobic treatment agent can be chosen from silicones, such as methicones, dimethicones or perfluoroalkylsilanes; fatty acids, such as stearic acid; metal soaps, such as aluminium dimyristate or the aluminium salt of hydrogenated tallow glutamate; perfluoroalkyl phosphates; perfluoroalkylsilanes; perfluoroalkylsilazanes; poly(hexafluoropropylene oxide)s; polyorganosiloxanes comprising perfluoroalkyl perfluoropolyether groups; amino acids; N-acylated amino acids or their salts; lecithin; isopropyl triisostearoyltitanate; and their mixtures.

The N-acylated amino acids can comprise an acyl group having 8 to 22 carbon atoms, such as, for example, a 2-ethylhexanoyl, caproyl, lauroyl, myristoyl, palmitoyl, stearoyl or cocoyl group. The salts of these compounds can be aluminium, magnesium, calcium, zirconium, zinc, sodium or potassium salts. The amino acid can be, for example, lysine, glutamic acid or alanine.

The term "alkyl" mentioned in the compounds cited above denotes in particular an alkyl group having from 1 to 30 carbon atoms, preferably having from 5 to 16 carbon atoms.

Hydrophobically treated pigments are described in particular in Application EP-A-1 086 683.

The pigments are present in the composition according to the invention in a content ranging from 1% to 30% by weight, with respect to the total weight of the composition, preferably ranging from 5% to 20% by weight.

In addition to the pigments, the composition according to the invention can comprise fillers and/or pearlescent agents.

The term "fillers" should be understood as meaning colourless or white and inorganic or synthetic particles of any shape which are insoluble in the medium of the composition, whatever the temperature at which the composition is manufactured.

The fillers can be inorganic or organic and of any shape, platelet, spherical or oblong, whatever the crystallographic form (for example, sheet, cubic, hexagonal, orthorhombic, and the like). Mention may be made of talc, mica, silica, kaolin, polyamide (Nylon®) powders, poly-β-alanine powders, polyethylene powders, polymethyl methacrylates, polyurethane powders, such as the powder formed from copolymer of hexamethylene diisocyanate and of trimethylol hexyllactone, sold under the names Plastic Powder D-400 by Toshiki, powders formed from tetrafluoroethylene polymers (Teflon®), lauroyl lysine, starch, boron nitride, polyvinylidene chloride/acrylonitrile copolymers, acrylic acid copolymers, silicone resin powders, in particular silsesquioxane powders (silicone resin powders described in particular in patent EP 293 795; Tospearls® from Toshiba, for example), polyorganosiloxane elastomer particles, precipitated calcium carbonate, magnesium carbonate, basic magnesium carbonate, hydroxyapatite, hollow silica microspheres, glass or ceramic microcapsules, metal soaps derived from organic carboxylic acids having from 8 to 22 carbon atoms, preferably from 12 to 18 carbon atoms, for example zinc stearate, magnesium stearate, lithium stearate, zinc laurate or magnesium myristate; barium sulphate, and their mixtures.

The fillers can be present in each of the compositions according to the invention in a total content ranging from 0.05% to 30% by weight, with respect to the total weight of the composition, preferably ranging from 0.5% to 15% by weight and preferentially ranging from 1% to 10% by weight.

In addition to the pigments and the fillers, the particulate phase of the invention can comprise pearlescent agents.

The term "pearlescent agents" should be understood as meaning iridescent particles, in particular produced by certain shellfish in their shells or also synthesized, which are insoluble in the medium of the composition.

Pearlescent agents can be chosen from white pearlescent pigments, such as mica covered with titanium oxide or with bismuth oxychloride, coloured pearlescent pigments, such as titanium oxide-coated mica with iron oxides, titanium oxide-coated mica with in particular ferric blue or chromium oxide or titanium oxide-coated mica with an organic pigment of the abovementioned type, and pearlescent pigments based on bismuth oxychloride.

The pearlescent agents can be present in each of the compositions according to the invention in a content ranging from 0.05% to 30% by weight, with respect to the total weight of the composition, preferably ranging from 1% to 20% by weight and preferentially ranging from 2% to 10% by weight.

The composition according to the invention can additionally comprise at least one water- or fat-soluble dye.

The term "fat-soluble dyes" should be understood as meaning compounds, generally organic compounds, which are soluble in fatty substances, such as oils.

The term "water-soluble dyes" should be understood as meaning compounds, generally organic compounds, which are soluble in water or water-miscible solvents.

The fat-soluble dyes are, for example, Sudan red, D&C Red No. 17, D&C Green No. 6, β-carotene, soybean oil, Sudan brown, D&C Yellow No. 11, D&C Violet No. 2, D&C Orange No. 5, quinoline yellow, annatto or bromo acids.

The water-soluble dyes, which are synthetic or natural, are, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper chlorophyllin, methylene blue, anthocyanins (oenocyanin, black carrot, hibiscus, elder), caramel or riboflavin.

Lecithin

The composition according to the invention can comprise a nonhydrogenated lecithin.

The latter is generally obtained by lipid extraction, using nonpolar solvents, from vegetable or animal fatty materials. This lipid fraction usually comprises predominantly glycerophospholipids, including phosphatidylcholine.

The animal or vegetable sources which can be used to extract nonhydrogenated lecithins are, for example, soybean, sunflower or eggs. The glycerophospholipids present in a high proportion in these lecithins are mainly phosphatidylcholine and phosphatidylethanolamine.

The nonhydrogenated lecithins suitable for the implementation of the present invention can be lecithins resulting from soybean, from sunflower or from eggs and/or their mixtures.

Lecithins are usually supplied in the form dissolved in fatty acids, triglycerides or other solvents, or in the form of powders or cakes.

These are usually mixtures of lecithins, the glycerophospholipid content of which, in the products as marketed, generally varies from approximately at least 15% to approximately at least 95%.

Mention may be made, among nonhydrogenated lecithins which may be suitable for the use of the cosmetic compositions in accordance with the present invention, of the lecithins sold under the references Nattermann Phospholipid®, Phospholipon 80® and Phosale 75® by American Lecithin Company and Epikuron 145V, Topcithin 300, Emulmetik 930 and Ovothin 200 by Lucas Meyer.

The nonhydrogenated lecithin can be present in the composition according to the invention in a content ranging from 0.01 to 10% by weight, with respect to the total weight of composition, preferably from 0.1 to 5% by weight and better still from 0.1 to 1% by weight.

**Composition expanded in volume**

The compositions according to the invention can, according to a preferred embodiment, be expanded in volume.

The compositions expanded in volume according to the invention are formed in a stable manner in the foam form using a base composition and air or an inert gas.

The air or inert gas can represent in particular from 10 to 500% of the volume of the composition not expanded in volume, preferably from 30 to 200%, more preferably from 35 to 150% and better still from 40 to 140%.

This volume can be calculated by comparing the density of the base composition and of the composition expanded in volume.

This volume corresponds to the degree of expansion in volume of the composition (or level of gas introduced into the composition) and can in particular be calculated by the following formula: Degree of expansion in volume

$$= \frac{d_{\text{before expanding in volume}} - d_{\text{foam}}}{d_{\text{foam}}} \times 100$$

Without implied limitation, this degree of expansion in volume can in particular be measured on a composition already expanded in volume according to the following protocol:

> The composition expanded in volume is introduced into a 100 ml beaker, so as to occupy the total volume thereof, while avoiding, during the filling of the jar, the formation of air bubbles.

The mixture is left standing for 10 min and then the volume V1, corresponding to the volume of the composition expanded in volume, is recorded.

A turbine is introduced and the beaker and the turbine are placed under a vacuum bell jar.

Vacuum is applied and the blade of the turbine is rotated at a slow speed (500 rev/min) for 10 min.

The vacuum is disconnected and the volume V2, corresponding to the volume of the base composition not expanded in volume, is recorded.

The degree of expansion in volume is subsequently calculated from the following formula:

$$\text{Degree of expansion in volume} = \frac{V_1 - V_2}{V_2} \times 100$$

In addition to the air, the gases which make it possible to obtain the composition expanded in volume are in particular inert gases, for example nitrogen, carbon dioxide, nitrogen oxides, noble gases or a mixture of the said gases. When the composition comprises an oxidation-sensitive compound, it is preferable to use an oxygen-free gas, such as nitrogen, or carbon dioxide.

The base composition which is used to obtain the composition expanded in volume has a composition similar to the composition expanded in volume, except for its higher density, insofar as it is devoid of air or inert gas. The amount of gas introduced into the base composition contributes to the adjustment to the desired value of the density of the composition expanded in volume.

The composition expanded in volume of the invention can exhibit a density of less than 0.95 $g/cm^3$. The composition expanded in volume of the invention advantageously has a density ranging from 0.2 to 0.9 $g/cm^3$ and preferably from 0.3 to 0.8 $g/cm^3$, this density being measured at a temperature of approximately 20°C and at atmospheric pressure, according to the following protocol.

The test is carried out on a volume of composition of 25 ml introduced into a smooth Plexiglas® jar with a volume of 25 ml ($V_1$) defining a cylindrical filling space with a height of 15 mm and a base with a diameter of 46 mm. The jar has a bottom wall with a thickness of 10 mm and a side wall with a thickness of 12 mm.

Prior to the measuring, the composition to be characterized and the jar are maintained at a temperature of the order of 20°C. The jar is tared and the weight value is noted ($W_1$). The composition expanded in volume is subsequently introduced into the jar so as to occupy the total volume thereof, while avoiding, during the filling of the jar, the formation of air bubbles. The combination is left standing at ambient temperature for 24 hours. The top of the jar is subsequently levelled before it is weighed ($W_2$). The density is evaluated according to the convention $\rho = (W_2 - W_1)/25$.

The composition expanded in volume according to the invention exhibits a satisfactory stability which can be calculated by measuring a volume of foam ($V_2$) remaining in the jar after one month at 45°C, according to the protocol described above for the measurement of the density.

The ratio $V_2/V_1$ corresponds to the ratio of the volume of the composition expanded in volume after one month at 45°C to the volume of the composition expanded in volume after 24 hours at ambient temperature.

The expression "satisfactory stability" applies in particular to compositions expanded in volume exhibiting a ratio $\frac{V_2}{V_1}$

of greater than 0.85, in particular of greater than 0.90, for example of greater than 0.95.

For a given weight of composition expanded in volume, the volume of the composition expanded volume is inversely proportional to the density of the composition expanded in volume. Thus, the ratio of the density of the composition expanded in volume, measured after 24 hours, to the density of the composition expanded in volume, measured after one month at 45°C, can be greater than 0.85, in particular greater than 0.90, for example greater than 0.95.

**Additional cosmetic ingredients**

The composition can also comprise other ingredients (adjuvants) conventionally used in cosmetics, such as, for example, preservatives, cosmetic active principles, moisturizing agents, UV screening agents and/or fragrances.

Of course, a person skilled in the art will take care to choose the optional adjuvant or adjuvants added to the composition according to the invention so that the advantageous properties intrinsically attached to the composition in accordance with the invention are not, or not substantially, detrimentally affected by the envisaged addition.

The composition according to the invention can in particular be provided in the form of a makeup product, in particular a foundation, a concealer or a product for making up the body, or alternatively in the form of a care product for the face or of a care product for the body.

The invention is illustrated in more detail in the following examples.

**Examples:**

A foundation was prepared which has the following composition:

|  |  |  | Composition 1 (% by weight) |
|---|---|---|---|
| Phase A1 | Deionized water | | q.s. for 100 |
| | Preservatives | | 0.5 |
| Phase A2 | Purified smectite | | 0.38 |
| | Xanthan gum | | 0.13 |
| | Hydrophobically modified inulin | | 0.2 |
| Phase A3 | Titanium dioxide | | 8.01 |
| | Yellow iron oxide | | 1.19 |
| | Red iron oxide | | 0.57 |
| | Black iron oxide | | 0.23 |
| | Deionized water | | 5.00 |
| | Glycerol | | 5.00 |
| Phase B | Triglycerides of caprylic and capric acid (60/40) | | 10.00 |
| | Jojoba liquid wax | | 10.00 |
| | Mixture of sorbitan mono- and distearate | | 2.00 |
| | Mixture of sorbitan mono/distearate and of potassium stearate | | 3.00 |
| | Preservatives | | 0.15 |
| Phase C | Deionized water | | 1.00 |
| | Preservative | | 0.25 |

Procedure:

The constituents of phase A1 were weighed out, heating being carried out at a temperature of 75°C while stirring using a Moritz mixer at 600 rev/min for 20 minutes.

The constituents of phase A2 were mixed and then A2 was added to A1.

Separately, the constituents of phase A3 were weighed out and ground on a triple roll mill and grinding was carried out until a homogeneous dispersion of fine particles, with a size approximately of less than 3 $\mu$m, was obtained.

A3 was added to the A1+A2 mixture while stirring with a Moritz mixer at 800 rev/min, the temperature being maintained at 65°C over 20 minutes.

The constituents of phase B were weighed out while heating at a temperature of 75°C while stirring with a magnetic bar.

B was added to the A1+A2+A3 mixture while stirring with a Moritz mixer at 1700 rev/min over 15 minutes.

The constituents of phase C were weighed out and then C was added to the B+A1+A2+A3 mixture while stirring with a Moritz mixer at 1700 rev/min over 5 minutes.

A second composition devoid of hydrophobically modified inulin was prepared which has the following composition:

| | | Composition 2 (% by weight) |
|---|---|---|
| Phase A1 | Deionized water | q.s. for 100 |
| | Preservatives | 0.5 |
| Phase A2 | Purified smectite | 0.38 |
| | Xanthan gum | 0.13 |
| Phase A3 | Titanium dioxide | 8.01 |
| | Yellow iron oxide | 1.19 |
| | Red iron oxide | 0.57 |
| | Black iron oxide | 0.23 |
| | Deionized water | 5.00 |
| | Glycerol | 5.00 |
| Phase B | Triglycerides of caprylic and capric acid (60/40) | 10.00 |
| | Jojoba liquid wax | 10.00 |
| | Mixture of sorbitan mono- and distearate | 2.00 |
| | Mixture of sorbitan mono/distearate and of potassium stearate | 3.00 |
| | Preservatives | 0.15 |
| Phase C | Deionized water | 1.00 |
| | Preservative | 0.25 |

Composition 2 is prepared according to the same procedure as Composition 1.

The viscosities of the two compositions, after storing at 20°C for 24 h, were measured on a Rheomat RM180 device with the following settings:

- Measurement system: 75
- Shear rate: 200 $s^{-1}$
- Jar with a diameter of 32.4 mm
- Spindle No. 3

The measurements are carried out at t(0) and then at t (10 min), that is to say after shearing for 10 minutes in a bath thermostatically controlled at 20°C.

A result expressed in "deflection unit" is obtained, which result is converted into poises using tables supplied with the device.

The following results are obtained.

| | Composition 1 with hydrophobically modified inulin | Composition 2 without hydrophobically modified inulin |
|---|---|---|
| Viscosity without prior shearing | 16.6 poises (+/- 0.5 poises) | 25.7 poises (+/- 0.5 poises) |
| Viscosity after shearing for 10 minutes | 10.9 poises (+/- 0.5 poises) | 17.7 poises (+/- 0.5 poises) |

Composition 1 was subsequently expanded in volume using a Kenwood Chef KM 300 electric beater with a power of 650 watts and with a capacity of 6.8 litres, including a working capacity of 5 litres.

## EP 2 146 680 B1

300 g of base composition are introduced into the bowl and the volume is expanded at ambient temperature for 20 minutes.
A degree of expansion of 87% is obtained.
A composition is obtained in the form of a foam which is stable at 45°C for 1 month.

### Claims

1. Cosmetic composition expanded in volume, in the form of an oil-in-water emulsion, comprising an internal fatty phase and an external aqueous phase comprising at least one inulin modified by hydrophobic chains and at least two gelling agents for the aqueous phase, one of the two gelling agents being chosen from unmodified clays, in particular montmorillonites, hectorites, smectites, bentones or laponites, or cellulose polymers, such as hydroxyethylcellulose, hydroxypropylcellulose, methylcellulose, ethylhydroxyethylcellulose, carboxymethylcellulose and quaternized cellulose derivatives, and the other gelling agent being chosen from gums arabic, guar gum, xanthan derivatives, karaya gum or locust bean gum, the said internal fatty phase being present in a content ranging from 5 to 50% by weight, wherein the composition is formed in a stadle manner in the foam form using a base composition and air using base composition and air or an inert gas.

2. Composition according to the preceding claim, **characterized in that** one of the two gelling agents is chosen from unmodified clays, in particular montmorillonites, hectorites, smectites, bentones or laponites, and the other gelling agent is chosen from gums arabic, guar gum, xanthan derivatives, karaya gum or locust bean gum.

3. Composition according to either one of the preceding claims, **characterized in that** the two gelling agents are xanthan gum and smectite.

4. Composition according to any one of the preceding claims, **characterized in that** it exhibits an internal fatty phase in a content ranging from 5 to 50% by weight, preferably from 10 to 40% by weight, more preferably from 15 to 30% by weight.

5. Composition according to any one of the preceding claims, **characterized in that** it comprises at least one wax in a content ranging from 1 to 15% by weight, preferably from 2 to 12% by weight and more preferably from 4 to 9% by weight, with respect to the total weight of the composition.

6. Composition according to either one of the preceding claims, **characterized in that** the hydrophilic gelling agents are present in a content on a dry basis ranging from 0.01% to 30% by weight, with respect to the total weight of the composition, preferably from 0.05% to 20% by weight, better still from 0.1% to 10% by weight and even better still from 0.1% to 10% by weight.

7. Composition according to any one of the preceding claims, **characterized in that** it comprises a pulverulent phase in a content ranging from 1 to 30% by weight, with respect to the total weight of the composition, preferably from 5 to 25% by weight, with respect to the total weight of the composition.

8. Composition according to any one of the preceding claims, **characterized in that** the inulin is obtained from endive.

9. Composition according to any one of the preceding claims, **characterized in that** the hydrophobic chains are saturated or unsaturated and linear or branched hydrocarbon chains having from 1 to 50 carbon atoms, such as alkyl, arylalkyl, alkylaryl or alkylene groups; divalent cycloaliphatic groups or organopolysiloxane chains comprising one or more ester, amide, urethane, carbamate, thiocarbamate, urea, thiourea and/or sulphonamide functional groups, such as in particular methylenedicyclohexyl and isophorone; or divalent aromatic groups, such as phenylene.

10. Composition according to any one of the preceding claims, **characterized in that** the inulin exhibits a degree of polymerization of 2 to approximately 1000 and preferably of 2 to approximately 60 and a degree of substitution of less than 2 on the basis of one fructose unit.

11. Composition according to any one of the preceding claims, **characterized in that** the hydrophobic chains exhibit at least one alkylcarbamate group of formula R-NH-CO- in which R is an alkyl group having from 1 to 22 carbon atoms.

12. Composition according to any one of the preceding claims, **characterized in that** the hydrophobic chains are laurylcarbamate groups.

13. Composition according to any one of the preceding claims, **characterized in that** the inulin is present in a content ranging from 0.01 to 20% by weight, with respect to the total weight of the composition, preferably from 0.05 to 15% by weight, more preferably from 0.1 to 5% by weight and more preferably still from 0.1 to 1% by weight.

14. Composition according to any one of the preceding claims, **characterized in that** it comprises a nonhydrogenated lecithin resulting from soybean, from sunflower or from eggs and/or their mixtures.

15. Method for making up and/or for caring for keratinous substances, comprising the application, to the said keratinous substances, of at least one composition according to any one of Claims 1 to 14.

**Patentansprüche**

1. Volumenexpandierte kosmetische Zusammensetzung in Form einer Öl-in-Wasser-Emulsion, umfassend eine interne Fettphase und eine externe wäßrige Phase, umfassend mindestens ein durch hydrophobe Ketten modifiziertes Inulin und mindestens zwei Geliermittel für die wäßrige Phase, wobei eines der beiden Geliermittel unter unmodifizierter Tonen, insbesondere Montmorilloniten, Hectoriten, Smektiten, Bentonen oder Laponiten, oder Cellulosepolymeren, wie Hydroxyethylcellulose, Hydroxypropylcellulose, Methylcellulose, Ethylhydroxyethylcellulose, Carboxymethylcellulose und quaternisierten Cellulosederivaten, ausgewählt ist und das andere Geliermittel unter Gummi arabicum, Guar-Gummi, Xanthanderivaten, Karaya-Gummi oder Johannisbrotgummi ausgewählt ist, wobei die interne Fettphase in einem Gehalt von 5 bis 50 Ges.-% vorliegt, wobei die Zusammensetzung unter Verwendung von einer Grundzusammensetzung und Luft oder einem Inertgas in stabiler Weise in Schaumform gebildet wird.

2. Zusammensetzung nach dem vorhergehenden Anspruch, **dadurch gekennzeichnet, daß** eines der beiden Geliermittel unter unmodifizierten Tonen, insbesondere Montmorilloniten, Hectoriten, Smektlten, Bentonen oder Laponiten, ausgewählt ist und das andere Geliermittel unter Gummi arabicum, Guar-Gummi, Xanthanderivaten, Karaya-Gummi oder Johannisbrotgummi ausgewählt ist.

3. Zusammensetzung nach einem der vorhergehenden Ansprüche, **dadurch gekennzeichnet, daß** es sich bei den beiden Geliermitteln um Xanthangummi und Smektit handelt.

4. Zusammensetzung nach einem der vorhergehenden Ansprüche, **dadurch gekennzeichnet, daß** sie eine interne Fettphase in einem Gehalt von 5 bis 50 Gew.-%, vorzugsweise 10 bis 40 Gew.-% und weiter bevorzugt 15 bis 30 Gew.-% aufweist.

5. Zusammensetzung nach einem der vorhergehenden Ansprüche, **dadurch gekennzeichnet, daß** sie mindestens ein Wachs in einem Gehalt von 1 bis 15 Ges.-%, vorzugsweise 2 bis 12 Gew.-% und weiter bevorzugt 4 bis 9 Gew.-%, bezogen auf das Gesamtgewicht der Zusammensetzung, umfaßt.

6. Zusammensetzung nach einem der vorhergehenden Ansprüche, **dadurch gekennzeichnet, daß** die hydrophilen Geliermittel in einem Gehalt auf Trockenbasis im Bereich von 0,01 bis 30 Gew.-%, bezogen auf das Gesamtgewicht der Zusammensetzung, vorzugsweise 0,05 bis 20 Gew.-%, noch besser 0,1 bis 10 Gew.-% und sogar noch besser 0,1 bis 10 Gew.-% vorliegen.

7. Zusammensetzung nach einem der vorhergehenden Ansprüche, **dadurch gekennzeichnet, daß** sie eine pulverförmige Phase in einem Gehalt von 1 bis 30 Gew.-%, bezogen auf das Gesamtgewicht der Zusammensetzung, und vorzugsweise 5 bis 25 Gew.-%, bezogen auf das Gesamtgewicht der Zusammensetzung, umfaßt.

8. Zusammensetzung nach einem der vorhergehenden Ansprüche, **dadurch gekennzeichnet, daß** das Inulin aus Endivien erhalten wird.

9. Zusammensetzung nach einem der vorhergehenden Ansprüche, **dadurch gekennzeichnet, daß** es sich bei den hydrophoben Ketten um gesättigte oder ungesättigte und lineare oder verzweigte Kohlenwasserstoffketten mit 1 bis 50 Kohlenstoffatomen, wie Alkyl-, Arylalkyl-, Alkylaryl- oder Alkylengruppen; zweiwertige cycloaliphatische Gruppen oder Organopolysiloxanketten mit einer oder mehreren Ester-, Amid-, Urethan-, Carbamat-, Thiocarbamat-, Harnstoff-, Thioharnstoff- und/oder Sulfonamidf-unktionen, wie insbesondere Methylendicyclohexyl und Isophoron;

oder zweiwertige aromatische Gruppen, wie Phenylen, handelt.

10. Zusammensetzung nach einem der vorhergehenden Ansprüche, **dadurch gekennzeichnet, daß** das Inulin einen Polymerisationsgrad von 2 bis ungefähr 1000 und vorzugsweise 2 bis ungefähr 60 und einen Substitutionsgrad von weniger als 2, bezogen auf eine Fructoseeinheit, aufweist.

11. Zusammensetzung nach einem der vorhergehenden Ansprüche, **dadurch gekennzeichnet, daß** die hydrophoben Ketten mindestens eine Alkylcarbamatgruppe der Formel R-NH-CO-, worin R für eine Alkylgruppe mit 1 bis 22 Kohlenstoffatomen steht, aufweisen.

12. Zusammensetzung nach einem der vorhergehenden Ansprüche, **dadurch gekennzeichnet, daß** es sich bei den hydrophoben Ketten um Laurylcarbamatgruppen handelt.

13. Zusammensetzung nach einem der vorhergehenden Ansprüche, **dadurch gekennzeichnet, daß** das Inulin einem Gehalt im Bereich von 0,01 bis 20 Gew.-%, bezogen auf das Gesamtgewicht der Zusammensetzung, vorzugweise 0,05 bis 15 Gew.-%, weiter bevorzugt 0,1 bis 5 Gew.-% und noch weiter bevorzugt 0,1 bis 1 Gew.-% vorliegt.

14. Zusammensetzung nach einem der vorhergehenden Ansprüche, **dadurch gekennzeichnet, daß** sie ein nichthydriertes Lecithin aus Sojabohnen, aus Sonnenblumen oder aus Eiern und/oder Mischungen davon umfaßt.

15. Verfahren zum Makeup und/oder zur Pflege von keratinhaltigen Substanzen, bei dem man auf die keratinhaltigen Substanzen mindestens eine Zusammensetzung nach einem der Ansprüche 1 bis 14 aufbringt.

## Revendications

1. Composition cosmétique foisonnée, sous forme d'émulsion huile-dans-eau, comprenant une phase grasse interne et une phase aqueuse externe comprenant au moins une inuline modifiée par des chaînes hydrophobes et au moins deux gélifiants de la phase aqueuse, l'un des deux gélifiants étant choisi parmi les argiles non modifiées et notamment les montmorillonites, les hectorites, les smectites, les bentones, les laponites ; les polymères de cellulose tels que l'hydroxyéthyilcellulose, l'hydroxypropylcellulose, la méthylcellulose, l'éthylhydroxyéthylcellulose, la carboxyméthylcellulose, ainsi que les dérivés quaternisés de la cellulose ; et l'autre gélifiant étant choisi parmi les gommes arabiques, la gomme de guar, les dérivés du xanthane, la gomme de karaya, la gomme de caroube, ladite phase grasse interne étant présente en une teneur allant de 5 à 50 % en poids, la composition étant formée de manière stable sous forme de mousse à l'aide d'une composition de base et d'air ou d'un gaz inerte.

2. Composition selon la revendication précédente, **caractérisée en ce que** l'un des deux gélifiants est choisi parmi les argiles non modifiées et notamment les montmorillonites, les hectorites, les smectites, les bentones, les laponites; et l'autre gélifiant est choisi parmi les gommes arabiques, la gomme de guar, les dérivés du xanthane, la gomme de karaya, la gomme de caroube.

3. Composition selon l'une quelconque des revendications précédentes, **caractérisée en ce que** les deux gélifiants sont la gomme de xanthane et la smectite.

4. Composition selon l'une quelconque des revendications précédentes, **caractérisée en ce qu'**elle présente une phase grasse interne en une teneur allant de 5 à 50 en poids, de préférence de 10 à 40 % en poids, de préférence encore de 15 à 30 % en poids.

5. Composition selon l'une quelconque des revendications précédentes, **caractérisée en ce qu'**elle comprend au moins une cire en une teneur allant de 1 à 15 en poids, de préférence de 2 à 12 % en poids, et de préférence encore de 4 à 9 % en poids, par rapport au poids total de la composition.

6. Composition selon l'une quelconque des revendications précédentes, **caractérisée en ce que** les gélifiants hydrophiles sont présents en une teneur en matières sèches allant de 0,01 % à 30 en poids par rapport au poids total de la composition, de préférence de 0,05 % à 20 % en poids, mieux de 0,1 % à 10 %, et mieux encore de 0,1 % 10 % en poids.

7. Composition selon l'une quelconque des revendications précédentes, **caractérisée en ce qu'**elle comprend une phase pulvérulente en une teneur allant de 1 à 30 % en poids, par rapport au poids total de la composition, de préférence de 5 à 25 % en poids, par rapport au poids total de la composition.

8. Composition selon l'une quelconque des revendications précédentes, **caractérisée en ce que** l'inuline est obtenue à partir de chicorée.

9. Composition selon l'une quelconque des revendications précédentes, **caractérisée en ce que** les chaînes hydrophobes sont des chaînes hydrocarbonées linéaires ou ramifiées, saturées ou insaturées, ayant de 1 à 50 atomes de carbone, telles que les groupements alkyle, arylalkyle, alkylaryle, alcoylène ; des groupements divalents cycloaliphatiques ou des chaînes organopolysiloxanes comprenant une ou plusieurs fonctions ester, amide, uréthane, carbamate, thiocarbamate, urée, thio-urée, et/ou sulfonamide tels que notamment méthylènedicyclohexyle et isophorone ; ou des groupements divalents aromatiques tels que phénylène.

10. Composition selon l'une quelconque des revendications précédentes, **caractérisée en ce que** l'inuline présente un degré de polymérisation de 2 à environ 1000 et de préférence de 2 à environ 60, et un degré de substitution inférieur à 2 sur la base d'une unité fructose.

11. Composition selon l'une quelconque des revendications précédentes, **caractérisée en ce que** les chaînes hydrophobes présentent au moins un groupement alkyle carbamate de formule R-NH-CO- dans laquelle R est un groupement alkyle ayant de 1 à 22 atomes de carbone.

12. Composition selon l'une quelconque des revendications précédentes, **caractérisée en ce que** les chaînes hydrophobes sont des groupements lauryle carbamate.

13. Composition selon l'une quelconque des revendications précédentes, **caractérisée en ce que** l'inuline est présente en une teneur allant de 0,01 à 20 % en poids par rapport au poids total de la composition, de préférence de 0,05 à 15 % en poids, plus préférentiellement de 0,1 à 5 % en poids, et plus préférentiellement encore de 0,1 à 1 % en poids.

14. Composition selon l'une quelconque des revendications précédentes, **caractérisée en ce qu'**elle comprend une lécithine non hydrogénée issue du soja, du tournesol, de l'oeuf et/ou de leurs mélanges.

15. Procédé de maquillage et/ou de soin des matières kératiniques comprenant l'application sur lesdites matières kératiniques d'au moins une composition selon l'une quelconque des revendications 1 à 14.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1396259 A
- US 5874069 A
- US 5919441 A
- US 6051216 A
- US 5981680 A
- EP 1086683 A
- EP 293795 A

**Non-patent literature cited in the description**

- *J. Soc. Cosm. Chem.,* 1954, vol. 5, 249-256
- **Kirk-Othmer.** Encyclopedia of Chemical Technology. Wiley, 1979, vol. 22, 333-432